# EUROPEAN PATENT APPLICATION

(11) **EP 2 014 650 A1**
(43) Date of publication of application: **14.01.2009**
(21) Application number: 07013810.2
(22) Date of filing: 13.07.2007
(51) Int. Cl.: C07D 215/18, C07D 405/10

(54) **Process and intermediate for the production of an intermediate in the production of montelukast**

(71) Applicant: Lonza AG, 4052 Basel (CH)
(72) Inventor: McGarrity, John, 3902 Brig-Glis (CH); Djojo, Francis, 3930 Visp (CH)

(57) **Abstract**

The tertiary alcohol α-[3-[(1*E*)2-(7-chloro-2-quinolinyl)ethenyl]phenyl]-2-(1-hydroxy-1-methylethyl)benzenepropanol, the (α*S*)-enantioner of which is an intermediate in the production of montelukast, is produced by reacting the novel lactone of formula 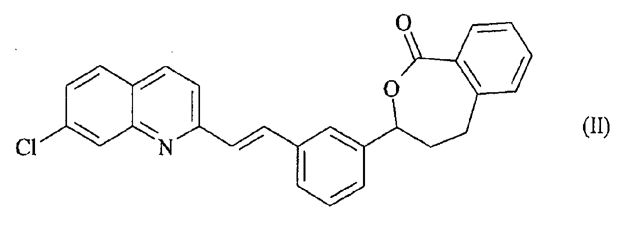 with a methylmagnesium halide in an ethereal solvent in the presence of lanthanum trichloride and lithium chloride. The lactone II can be prepared by reacting a corresponding hydroxycster with a Grignard reagent in the absence of a lanthanoid compound.

## Description

The invention relates to a process for the production of a tertiary alcohol of formula 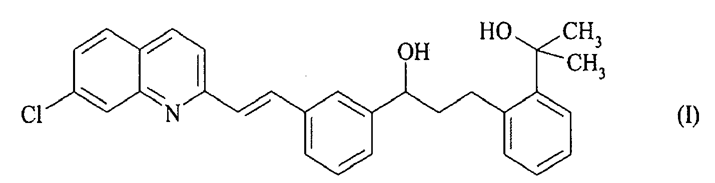 namely, *α*-[3-[(1*E*)-2-(7-chloro-2-quinolinyl)ethenyl]phenyl]-2-(1-hydroxy-1-methylethyl)-benzenepropanol, the (α*S*)-stereoisomer of which is a key intermediate in the synthesis of the pharmaceutically active compound known as montelukast (1-[[[(1*R*)*-*1-[3-[(1*E*)-2-(7-chloro-2-quinolinyl)ethenyl]phenyl]-3-[2-(1-hydroxy-1-methylethyl)phenyl]propyl]thio]methyl]-cyclopropaneacetic acid). In further relates to a new intermediate of said process and a method for its preparation.

A known synthesis of(α*S*)-α-[3-[(1*E*)-2-(7-chloro-2-quinolinyl)ethenyl]phenyl]-2-(1-hydroxy-1-methylethyl)benzenepropanol, is based on the reaction of a carboxylic ester with two equivalents of a Grignard reagent. However, the yields are not always satisfactory as undesired reactions compete with the formation of the alcohol and result in the formation of byproducts, in particular when an alkylmagnesium chloride is used as Grignard reagent (D. A. Conlon et al., Adv. Synth. Catal. 2004, 346, 1307-1315). It has been found that "nearly anhydrous" activated cerium trichloride has a beneficial effect on the above reaction, which has been postulated to be due to suppression of the enolization of the ketone intermediate. The water content and activation method of the cerium trichloride as well as its crystal habit have been found to be critical. Moreover, the activation of the cerium chloride is somewhat tedious and the activated cerium chloride is sparingly soluble in ethereal solvents such as tetrahydrofuran which results in a heterogeneous reaction mixture. In the preparation of the above montelukast intermediate the starting material (which is available as a monohydrate) has first to be carefully dried (e.g. by azeotropic distillation), but nevertheless, about 5 equivalents of methylmagnesium chloride are required, instead of the theoretical amount of 3 equivalents (WO 95/18107 A1).

It is an object of the present invention to provide an improved method for the preparation of the tertiary alcohol α-[3-[(1*E*)-2-(7-chloro-2-quinolinyl)ethenyl]phenyl]-2-(1-hydroxy-1-methylethyl)benzenepropanol from a corresponding carboxylic ester and a Grignard reagent which gives high yields of the desired product even if the chloride form of the Grignard reagent is used. The method should not involve tedious activation steps, heterogeneous reaction mixtures and cumbersome work-up procedures.

Applicants have found that the desired product is readily available by reacting the lactone of formula 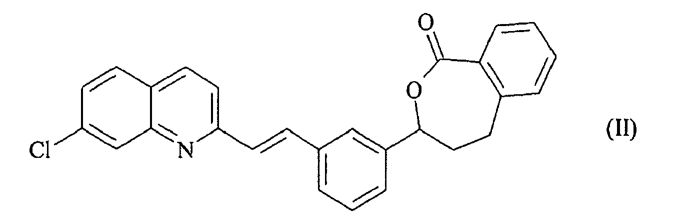 namely, 3-[3-[(*E*)-2-(7-chloro-2-quinolinyl)ethenyl]phenyl]-4,5-dihydro-3*H*-benzo[*c*]oxepin-1-one,
with a Grignard reagent of formula

CH₃MgX (III),

wherein X is chlorine, bromine or iodine,
in an ethereal solvent in the presence of lanthanum trichloride and lithium chloride.

The term "ethereal solvent" is to be understood to mean any solvent or solvent mixture comprising a substantial amount of an acyclic or cyclic ether that is liquid at the reaction temperature, such as diethyl ether, dibutyl ether, methyl *tert-*butyl ether, dimethoxyethane, tetrahydrofuran (THF), dioxane and the like.

The lithium chloride solubilizes the lanthanum trichloride, resulting in a true solution of the two salts in the ethereal solvent and thus in a homogeneous reaction mixture. In a preferred embodiment, lanthanum trichloride and lithium chloride are present in a molar ratio of 1:2 or less. A tetrahydrofuran solution of LaCl₃ and LiCl in a molar ratio of 1:2 is commercially available from Chemetall GmbH, Frankfurt (Main), Germany.

The halogen component X of the Grignard reagent III is preferably chlorine.

Most preferably, the secondary alcohol group and the carbon atom in position 3 of the oxepine ring, respectively, of the above structures I and II have *S*-configuration to make them suitable as intermediates in the synthesis of (R)-montelukast.

The ethereal solvent used in the process of the invention is preferably tetrahydrofuran alone or a mixture of tetrahydrofuran and an inert solvent such as an aliphatic or aromatic hydrocarbon.

The reaction temperature can be in the range that is commonly employed in Grignard reactions, it is preferably between -20 °C and room temperature, more preferably from -10°C to +10°C.

The work-up of the reaction mixture can be accomplished according to the methods commonly used in the art, e.g. by quenching with water or weak aqueous acids and extracting the product with a suitable solvent.

A particular advantage of the process according to the invention resides in the fact that, in contrast to the hydroxyester conventionally used as starting material (cf. WO 95/18107 A1), the lactone II has no active hydrogen that would result in the consumption of another equivalent of Grignard reagent. Another advantage resides in the fact that the amount of lanthanum chloride required is substantially lower than the amount of cerium chloride employed in the prior art process. The prior art process used cerium trichloride in a molar ratio of CeCl₃/hydroxyester starting material of about 1:1 while the process of the present invention can be carried out with substantially lower lanthanum trichloride/lactone ratios down to 1:4 or even less. This is especially advantageous in the work-up of the reaction mixture since the amount of magnesium and rare earth metal-containing wastes is quite substantially reduced.

The lactone of formula II is a novel compound and likewise an object of the invention.

In a preferred embodiment, the carbon atom in position 3 of the oxepine ring of the lactone II has *S*-configuration.

A further object of the invention is a process for the preparation of the lactone II. Applicants have surprisingly found that this compound is obtained in high selectivity when a carboxylic ester of formula 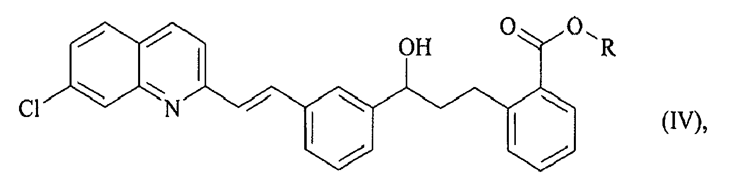 wherein R is C₁₋₁₀ alkyl, aryl or arylalkyl
with a Grignard reagent of formula

R¹MgCl (V)

wherein R¹ is C₁₋₄ alkyl,
in an ethereal solvent in the absence of a lanthanoid compound, such as cerium or lanthanum chloride.

The term "C_{1-*n*} alkyl" is here to be understood to comprise any linear or branched alkyl group having from 1 to n carbon atoms. For example, the tenn "C₁₋₄ alkyl" comprises methyl, ethyl, propyl, isopropyl, butyl, isobutyl, *sec*-butyl and *tert*-butyl. In addition to the beforementioned, the term "C₁₋₁₀ alkyl" comprises groups such as pentyl, isopentyl, neopentyl, hexyl, isohexyl, heptyl, octyl, nonyl, decyl and the like.

The term "aryl" is to be understood to comprise any mono-, bi- or polycarbocyclic group comprising at least one aromatic ring, such as phenyl, naphthyl, anthracenyl, phenanthryl, biphenylyl, fluorenyl, tetrahydronaphthalenyl and the like. A preferred meaning of "aryl" is phenyl.

The term "arylalkyl" is to be understood to comprise an alkyl group, and in particular a C₁₋₄ alkyl group, which is substituted with one of the groups mentioned above under "alkyl". The most preferred meaning of arylalkyl is benzyl.

In a preferred embodiment, the group R in the ester moiety is methyl.

More preferably, the methyl ester is employed in the form of its monohydrate without previous removal of its water of crystallization.

Both the carbon atom in position 3 of the oxepin ring in formula II and the secondary alcohol group in formula IV are preferably in the *S*-configuration.

The following non-limiting examples will illustrate the process of the invention.

### Example 1

### (3S)-3-[3-[(E)-2-(7-chloro-2-quinolinyl)ethenyl]phenyl]-4,5-dihydro-3H-benzo[c]oxepin-1-one

A suspension of methyl 2-[(3*S*)-3-[3-[(1*E*)-2-(7-chloro-2-quinolinyl)ethenyl]phenyl]-3-hydroxypropyl]benzoate monohydrate (18.32 g, 38.5 mmol) in 60 mL THF and 210 mL toluene was cooled to -5 °C in a 250 mL double-jacketed reactor under nitrogen. Methylmagnesium chloride (3 M solution in THF, 53.4 mL, 160 mmol) was added dropwise during 25 min while maintaining the temperature at -5 °C. The brown suspension was stirred at 0 °C for 4 h and at 20°C for 24 h. The reaction mixture was added during 15 min to 2 M aqueous acetic acid which had been pre-cooled to 5°C. During the addition the temperature rose to 12 °C. The mixture was stirred at this temperature for another 5 min, and the phases were separated. The aqueous phase was discarded and the organic phase was washed with 10% aqueous sodium carbonate solution, and then with 1% aqueous sodium carbonate solution (320 mL each). The solution was evaporated *in vacuo* (40°C, 30 mbar) to yield 24 g residue which was dissolved in THF (15 mL) at 45 °C. Heptane (41 mL) was added dropwise to this solution. The suspension formed was cooled to 0 °C, filtered, washed with heptane (30 mL) and dried to yield 8.57 g beige solid.
m.p.: 160 °C
IR (KBr): ṽ = 2931, 1714, 1608, 1497, 1455, 1410, 1297, 1251, 1121, 1084, 1039, 927, 875, 832, 799, 774, 755, 731, 693 cm⁻¹
¹H NMR (DMSO-d₆, 500 MHz): δ = 2.28 (m, 1H), 2.45 (m, 1H), 2.98 (m, 1H), 3.04 (m, 1H), 5.17 (dd, *J*= 12.2, 4.9 Hz; 1H), 7.45 (m, 3H), 7.49 (m, 1H), 7.52 (d, *J*= 16.6 Hz, 1H), 7.58 (dd, *J*= 8.8, 1.9 Hz; 1H), 7.63 (t, *J*= 7.5 Hz, 1H), 7.68 (m, 1H), 7.71 (d, *J*= 7.3 Hz, 1H), 7,85 (s, 1H), 7.87 (d, *J* = 16.6 Hz, 1H), 7.88 (d, *J* = 8.8 Hz, 1H), 7.98 (d, *J* = 8.8 Hz, 1H), 8.0 (d, *J*= 1.5 Hz, 1H), 8.38 (d, *J*= 8.8 Hz, 1H).
¹³C NMR (DMSO-d₆,126 MHz): δ = 29.24, 35.29, 78.86, 120.28, 125.20, 125.55, 126.61, 126.81, 127.16, 127.28, 128.71, 128.89,128.97,129.66,129.80,131.42,132.66,134.25,134.48, 136.20, 136.49, 137.60, 139.81, 147.96, 156.61, 170.01.

### Example 2

### (αS)-α-[3-[5(1E)-2-(7-chloro-2-quinolinyl)ethenyl]phenyl]-2-(1-hydroxy-1-methylethyl)-benzenepropanol

A solution of (3*S*)-3-[3-[(*E*)-2-(7-chloro-2-quinolinyl)ethenyl]phenyl]-4,5-dihydro-3*H-*benzo[*c*]oxepin-1-one (3.05 g, 7.2 mmol) in THF (45 mL) was cooled to 5 °C in a 250 mL double-jacketed reactor under nitrogen. Methylmagnesium chloride (3 M solution in THF, 7.11 g, 21.0 mmol) was added. Lanthanum chloride/lithium chloride (molar ratio 1:2, 16% solution in THF, 3.23 g, 1.6 mmol) was added with stirring at 5°C. The reaction mixture was heated to 20°C and stirred at that temperature for another 1 h. Completion of the reaction was ensured by HPLC control. The solution was cooled to below 10°C, and 2 M aqueous acetic acid (50 ml) was added during 5 min, followed by methyl *tert-*butyl ether (50 mL). The phases were separated and the organic phase was washed first with 10% aqueous sodium carbonate solution (50 mL) and then with saturated brine (50 mL). The organic phase was concentrated *in vacuo* (40 °C, 280 mbar) to a weight of 6.83 g. Heptane (17 mL) was added dropwise to this residue during 1 h at 25 °C, then the suspension was cooled to 0 °C and stirred for 1 h. The precipitate was filtered, washed with heptane (10 mL) and dried *in vacuo* at 30°C to yield 2.31 g light beige powder, purity (HPLC) 94.9%.
¹H NMR (DMSO-d₆, 500 MHz): δ = 1.51 (s, 3H); 1.52 (s, 3H); 2.00 (m, 2H), 2.96 (m, 1H); 3.10 (m, 1H); 4.72 (m, 1H); 4.94 (s, 1H); 5.36 (d, *J* = 4.4 Hz, 1H); 7.09 (t, *J* = 7.6 Hz, 1H); 7.14 (t, *J* = 7.8 Hz, I H); 7.18 (d, *J* = 6.4 Hz, 1H); 7.41 (m, 2H); 7.44 (d, *J* = 7.9 Hz, 1H); 7.49 (d, *J* = 16.6 Hz, 1H); 7.56 (dd, *J* = 8.3,2.2 Hz, 1H); 7.62 (d, *J* = 6.8 Hz, 1H); 7.77 (bs, 1H); 7.91 (d, *J* = 16.6 Hz, 1H); 7.92 (d, *J* = 8.7 Hz, 1H); 7.99 (d, *J* = 8.8 Hz, 1H); 8.03 (d, *J* = 2.0 Hz, I H); 8.38 (d, *J* = 8.4 Hz, I H).
¹³C NMR (DMSO-d6, 126 MHz): δ = 29.82, 31.55, 31.57, 42.34, 71.60, 72.31, 120.24, 124.73, 124.88, 125.24, 125.51, 125.71, 126.22, 126.54, 127.16, 128.04, 128.49, 129.65, 130.82, 134.23, 135.20, 135.67, 136.43, 140.25, 146.66, 146.93, 147.99, 156.78.

## Claims

1. A process for the production of [3-[(1*E*)-2-(7-chloro-2-quinolinyl)ethenyl]phenyl]-2-(1-hydroxy-1-methylethyl)benzenepropanol of formula 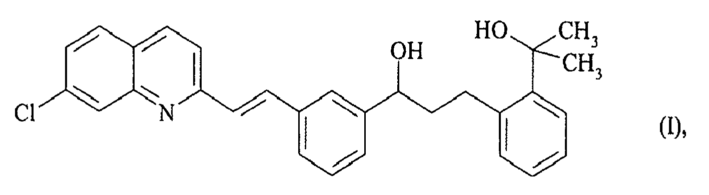 by reacting 3-[3-[(*E*)-2-(7-chloro-2-quinolinyl)ethenyl]phenyl]-4,5-dihydro-3*H*-benzo[*c*]-oxepin-1-one of formula 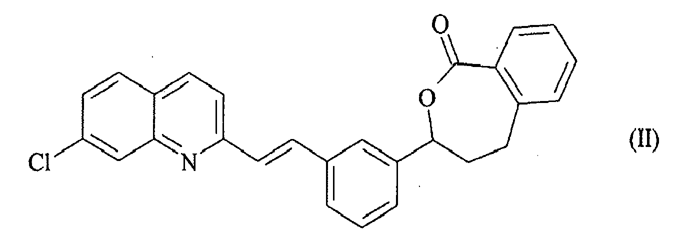 with a Grignard reagent of formula
CH₃MgX (III),
wherein X is chlorine, bromine or iodine,
in an ethereal solvent in the presence of lanthanum trichloride and lithium chloride.

2. The process of claim 1, wherein lanthanum trichloride and lithium chloride are present in a molar ratio of 1:2.

3. The process of claim 1 or 2, wherein X is chlorine.

4. The process of any of claims 1 to 3, wherein the secondary alcohol group in I and the carbon atom in position 3 of the oxepine ring have *S*-configuration.

5. The process of any of claim 1 to 4, wherein the ethereal solvent is tetrahydrofuran or a mixture of tetrahydrofuran and an inert solvent.

6. 3-[3-[(*E*)-2-(7-chloro-2-quinolinyl)ethenyl]phenyl]-4,5-dihydro-3*H*-benzo[*c*]oxepin-1-one of formula

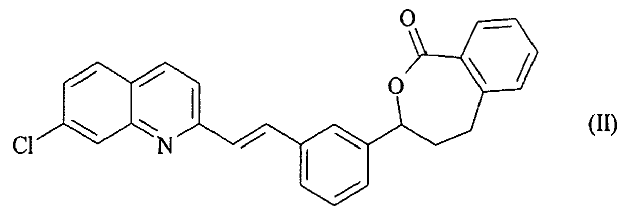

7. The compound of claim 6, which is (3*S*)-3-[3-[(*E*)-2-(7-chloro-2-quinolinyl)ethenyl]-phenyl]-4,5-dihydro-3*H*-benzo[*c*]oxepin-1-one of formula

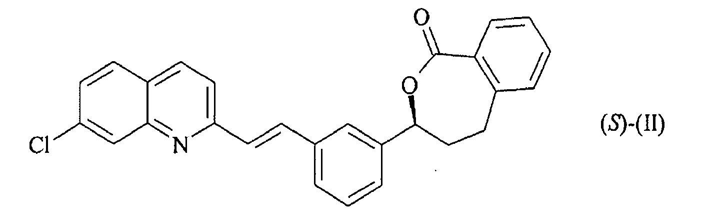

8. A process for the preparation of 3-[3-[(*E*)-2-(7-chloro-2-quinolinyl)ethenyl]phenyl]-4,5-dihydro-3*H*-benzo[*c*]oxepin-1-one of formula 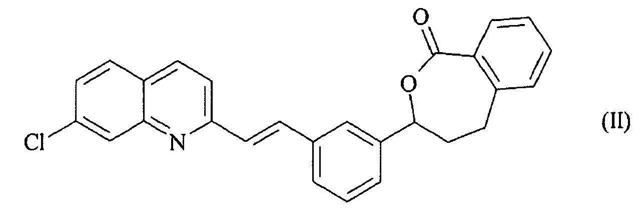 comprising reacting a carboxylic ester of formula 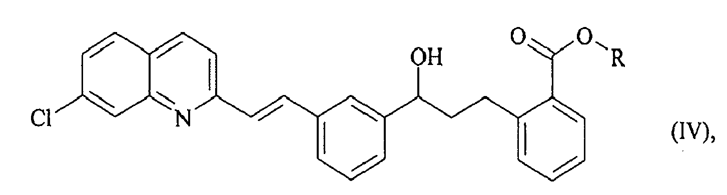 wherein R is C₁₋₁₀ alkyl, aryl or arylalkyl
with a Grignard reagent of formula
R¹ MgCl (V)
wherein R¹ is C₁₋₄ alkyl,
in an ethereal solvent in the absence of a lanthanoid compound.

9. The process of claim 8, wherein R is methyl.

10. The process of claim 9, wherein the carboxylic ester IV is in the monohydrate form.

11. The process of any of claims 8 to 10, wherein R¹ is methyl.

12. The process of any of claims 8 to 11, wherein the carbon atom in position 3 of the oxepin ring in formula II and the secondary alcohol group in formula IV have S-confguration.

13. The process of any of claims 8 to 12, wherein the ethereal solvent is tetrahydrofuran or a mixture of tetrahydrofuran and an inert solvent.
